# EUROPÄISCHE PATENTANMELDUNG

(11) **EP 0 550 836 A2**
(43) Veröffentlichungstag der Anmeldung: **14.07.1993**
(21) Anmeldenummer: 92120870.8
(22) Anmeldetag: 08.12.1992
(51) Int. Cl.: A61L 2/04, A23L 3/22

(54) **Heisssterilisierung**

(30) Priorität: 10.01.1992 DE 4200588
(71) Anmelder: KRUPP MASCHINENTECHNIK GESELLSCHAFT MIT BESCHRÄNKTER HAFTUNG, D-45143 Essen (DE)
(72) Erfinder: Gollasch, Rainer, Dipl.-Ing., W-2000 Hamburg 50 (DE); Hasenbein, Günter, Dipl.-Ing., W-2077 Grossensee (DE); Krause, Wolfgang, Dipl.-Ing., W-2093 Stelle (DE); Sitzmann, Werner, Dr.-Ing., W-2100 Hamburg 90 (DE)

(57) **Zusammenfassung**

Die Sterilisation pumpfähigen Behandlungsgutes im Heißsterilisierungsverfahren stößt insbesondere dann auf Schwierigkeiten, wenn sie im Durchlaufbetrieb und gleichzeitig zuverlässig und wirtschaftlich erfolgen soll. Deshalb wird das Behandlungsgut zunächst unter Sattdampfdruck gesetzt, auf die Sterilisationstemperatur erhitzt und von inerten Gasen befreit und dann über eine ausreichende Sterilisationszeit während der Durchleitung durch einen Sterilisationsbehälter auf der Sterilisationstemperatur gehalten. Ausreichende Sterilisationszeit bedeutet dabei, daß jedes Teilchen sicher sterilisiert, aber möglichst nicht länger als notwendig behandelt wird, also ein kleines Verweilzeitspektrum bezüglich der Sterilisationsbedingungen vorliegt. Dazu erfolgt der Durchlauf des Behandlungsgutes durch den Sterilisationsbehälter in mehreren in Durchlaufrichtung aufeinanderfolgenden Teilmengen, die durch gesättigten Wasserdampf voneinander getrennt sind. Der Sterilisationsbehälter ist als um ihre Längsachse drehbar angetriebene Rohrwendel (12) ausgebildet und Bestandteil von durch einen Behältermantel (9) umfaßten drei in Längsrichtung aufeinanderfolgenden Abschnitten, nämlich einer Aufgabekammer (11), der daran angeschlossenen Rohrwendel (12) und einer Entleerungskammer (13), wobei die zugeführte Behandlungsgutmenge und die Drehgeschwindigkeit so aufeinander abgestimmt sind, daß nur die jeweils unten liegende Hälfte der Rohrwendel (12) mit Behandlungsgut gefüllt ist.

## Beschreibung

Die Erfindung bezieht sich auf ein Verfahren zur Sterilisation von pumpfähigem Behandlungsgut durch Aufheizen, Durchleiten durch einen beheizten liegenden, Sterilisationsbehälter und anschließendes Entspannen und Abkühlen des Behandlungsgutes sowie eine Vorrichtung zur Durchführung dieses Verfahrens.

Besonders bei Produkten, die für den menschlichen Verzehr oder zur Tierernährung hergestellt werden, muß gewährleistet sein, daß sie eine möglichst lange Lagerfähigkeit aufweisen und eine Gesundheitsgefährdung der Verbraucher ausgeschlossen wird. Wirtschaftliche Aspekte spielen besonders in der Lebens- und Futtermittelindustrie, in der große Produktmengen mit vergleichsweise geringer Wertschöpfung verarbeitet werden, eine große Rolle. Im Vergleich zu chemischen Verfahren und Bestrahlungsmethoden ist die Hitze-Sterilisation das am häufigsten angewandte Behandlungsverfahren, wobei unter Sterilisation definitionsgemäß die Abtötung oder Entfernung von Mikroorganismen und die Inaktivierung von Viren, die sich in einem Produkt befinden, zu verstehen ist.

Diskontinuierliche Verfahren haben den Vorteil, daß hier die Sterilisationsbedingungen hinsichtlich Verweilzeit und Temperatur sehr genau eingehalten werden können. Als Apparaturen werden häufig relativ einfache Rührwerksautoklaven eingesetzt. Der Nachteil besteht darin, daß beim absatzweisen Betrieb durch Befüllen, Entleeren, Aufheizen und Abkühlen bzw. Entspannen viel Zeit und thermische Energie verlorengeht, was diese Verfahren besonders für die Verarbeitung großer Mengenströme unwirtschaftlich werden läßt. Ferner steigen die Investitionskosten mit den zu verarbeitenden Mengenströmen nahezu linear an.

Bislang bekannte kontinuierlich arbeitende Sterilisationsverfahren eliminieren zwar diese Nachteile, haben aber ihrerseits den gravierenden Nachteil, daß es bislang nur unzureichend gelang, die Sterilisationsbedingungen einzuhalten.

In der französischen Patentschrift 1 486 467 wird ein kontinuierlich arbeitendes Verfahren, bestehend aus den Schritten Aufheizung, Sterilisation und Entspannung beschrieben, wobei diese drei Teilschritte in indirekt beheizten, liegenden mit auf einer Welle angeordneten Rühr-/Förderorganen versehenen, zylindrischen Apparaten durchgeführt werden. Die Förderung des Materials soll dabei mittels Förderschnecken erfolgen, wobei die Abdichtung des druckbeaufschlagten Sterilisationsapparates zu den jeweils vor- und nachgeschalteten druckfreien Apparaten bzw. Verfahrensschritten mit Hilfe zweier perforierter Platten erfolgen soll, die am Eingang und am Ausgang des Sterilisationsbehälters angebracht sind, durch deren Lochung - nach dem Extruder- bzw. Expanderprinzip - das Material mit Hilfe der oben erwähnten Förderschnecken transportiert wird. Dabei wird davon ausgegangen, daß die in den Lochungen der perforierten Platten geformten Preßlinge eine druckstabile und gasdichte Sperrstrecke darstellen.

Die Erfahrung zeigt allerdings, daß besonders bei der Verarbeitung von feststoffhaltigen Materialien, wie z.B. von tierischen Abfällen, Schlachtnebenprodukten, Fleischknochen-Massen oder ähnlichem derartige Lochplatten, vor allem bei der häufig vorkommenden Anwesenheit produktfremder Feststoffe (Metalle, Kunststoffe etc.), außerordentlich schnell zur Verstopfung neigen. Außerdem ist, bedingt durch die Bauart des Sterilisators, mit einer breiten Verweilzeitverteilung des zu sterilisierenden Materials zu rechnen. Die kontrollierbare Einhaltung einer bestimmten Mindestverweilzeit im Sterilisator ist kaum realisierbar, da die Verweilzeitverteilung innerhalb des Sterilisators vom Mischungsverhalten und damit von der jeweiligen Zusammensetzung des Materials abhängt.

In der australischen Patentschrift 464 080 wird im Gegensatz dazu eine Apparatur zur Sterilisation von Tierfutter animalischen Ursprungs vorgestellt, bei der zur Aufheizung des zu behandelnden Materials Direktdampf über Düsen, die im Inneren eines materialdurchströmten Rohres angebracht sind, zugegeben wird. Wenngleich durch diese Maßnahme ein schnelleres und auch schonenderes Aufheizen des Materials realisierbar ist, besitzt diese Vorgehensweise den gravierenden Nachteil, daß das anfallende Kondensat in einer nachfolgenden Trocknungsstufe wieder entfernt werden muß. Außerdem erscheint die Zuführung des Dampfes über Düsen, die eine Querschnittsverengung innerhalb des materialdurchströmten Rohres zur Folge haben, vor allem bei der Behandlung von feststoffhaltigen Materialien wegen der vorhandenen Verstopfungsgefahr nicht günstig. Der entscheidende Nachteil dieses Verfahrens liegt allerdings darin, daß die Sterilisation selbst in einem vertikal angeordneten, von unten nach oben durchströmten, zylindrischen Gefäß erfolgt. Eine definierte Verweilzeit aller Volumenelemente im Inneren eines so ausgebildeten Sterilisationsbehälters ist aufgrund der auftretenden Stoffvermischung in horizontaler, vor allem aber in vertikaler Richtung nicht gegeben.

Einfache Rohrsterilisatoren gemäß der europäischen Veröffentlichungsschrift 0 338 615 sind allenfalls für sehr dünnflüssige und einphasige Medien einzusetzen, bei denen sich schon bei geringen Fließgeschwindigkeiten eine turbulente Rohrströmung ausbildet, die wegen des abgeflachten Strömungsprofils der idealen Kolbenströmung sehr nahe kommt. Die Turbulenz bewirkt auch eine gleichmäßigere Temperaturverteilung über den Rohrquerschnitt. Bei dickflüssigen und mehrphasigen Einsatzmaterialien (z.B. Fleischbrei) ist die exakte Einhaltung der Sterilisationszeit für alle Partikel jedoch aufgrund der axialen Längsvermischung für Laminar- und Mehrphasenströmung im Strömungsrohr nicht gewährleistet. Ferner ist die Temperaturverteilung über den Rohrquerschnitt inhomogen, was eine Überhitzung und Schädigung des Materials im Wandbereich bei gleichzeitiger Unterschreitung der erforderlichen Sterilisationstemperatur im Inneren des Rohres zur Folge haben kann. Darüberhinaus ist ein Entgasungsschritt nicht vorgesehen. Dies hat zur Folge, daß Inertgase, die von vornherein im Material enthalten sind bzw. während der Aufheiz- und Sterilisationsphase entstehen und freigesetzt werden, als Gaspolster das Rohrsystem durchwandern. Dadurch ergibt sich zwangsläufig eine Verschlechterung des Wärmeübertrages in das zu behandelnde Material. Außerdem ist denkbar, daß Mikroorganismen, die sich innerhalb oder auf der Grenzfläche dieser Gaspolster befinden, nicht in der vorgegebenen Sterilisationszeit abgetötet werden.

Es ist Aufgabe der vorliegenden Erfindung, diese Nachteile zu vermeiden und eine zuverlässige und kostengünstige Sterilisation anzubieten. Danach wird bei einem Verfahren der eingangs angegebenen Art vorgeschlagen, daß das Behandlungsgut zunächst unter mindestens den Sattdampfdruck, bezogen auf seine Sterilisationstemperatur, gesetzt, dann auf mindestens die Sterilisationstemperatur erhitzt und von inerten Gasen befreit und danach über eine ausreichende Sterilisationszeit während der Durchleitung durch den Sterilisationsbehälter auf mindestens der Sterilisationstemperatur gehalten wird. Ausreichende Sterilisationszeit bedeutet dabei, daß jedes Teilchen sicher sterilisiert, aber möglichst nicht länger als notwendig behandelt wird, also ein kleines Verweilzeitspektrum bezüglich der Sterilisationsbedingungen vorliegt. Dadurch wird eine sehr gleichmäßige, von Störungen durch Inertgase freie und zuverlässige Sterilisation erreicht.

Zweckmäßig erfolgt die Aufheizung in zwei Stufen, nämlich einer Vorwärmstufe, in der anfallende Abwärme genutzt, und einer Enderhitzerstufe, in der aus Primärenergie erzeugte Wärme verwendet wird. Dabei kann beispielsweise die Abwärme aus der Entspannung des Behandlungsgutes genutzt werden.

Zur weiteren Verbesserung der Wirtschaftlichkeit kann auch die Vorwärmung in zwei Schritten erfolgen, wobei im ersten Schritt bei der Entspannung anfallender Wasserdampf und im zweiten Schritt Abwärme aus der Enderhitzerstufe eingesetzt wird.

Zusätzlich wird empfohlen, daß die Enderhitzung in der Weise erfolgt, daß elektrischer Strom unmittelbar durch das Behandlungsgut fließt und dieses infolge seines ohmschen Widerstandes erwärmt, um auf diese Weise eine ausgezeichnete Temperaturverteilung innerhalb des Behandlungsgutes zu erzielen.

Vorteilhaft wird das Behandlungsgut aus der Vorwärmstufe kontinuierlich in einen Vorlagebehälter gegeben und von dort mittels einer Pumpe über den Enderhitzer im Kreislauf zum Vorlagebehälter zurückgeführt, wobei im Vorlagebehälter die Entgasung erfolgt. Damit ist einmal die Inertgasentfernung bereits vor dem Eintritt des Behandlungsgutes in den Sterilisationsbehälter gewährleistet. Zum anderen kann das Behandlungsgut auch kontinuierlich in die Vorwärmstufe eingetragen und das Verfahren in seiner Gesamtheit als kontinuierlich eingesetzt werden.

Zur Fortsetzung dieser kontinuierlichen Verfahrensführung wird aus dem Kreislauf zwischen Enderhitzer und Vorlagebehälter kontinuierlich ein Teilstrom des Behandlungsgutes abgezogen und dem Sterilisationsbehälter zugeführt, der dem aus der Vorwärmstufe in den Kreislauf eingeführten Mengenstrom entspricht, wobei der im Kreislauf geführte Mengenstrom ein Mehrfaches hiervon beträgt. Das Verhältnis der Mengenströme erlaubt eine gleichmäßige und wirksame, gleichzeitig auch sparsame Aufheizung.

Darüber hinaus ist vorgesehen, daß die Entgasung in der Weise erfolgt, daß im Gasraum des Vorlagebehälters eine wasserdampfgesättigte Atmosphäre vorliegt. Auch insoweit ist eine besonders günstige Verfahrensschrittfolge gegeben.

Gemäß einem weiteren Schritt der Erfindung erfolgt der Durchlauf des Behandlungsgutes durch den Sterilisationsbehälter in mehreren in Durchlaufrichtung aufeinanderfolgenden Teilmengen, die je voneinander durch gesättigten Wasserdampf getrennt sind. Diese Behandlung ermöglicht, innerhalb eines insgesamt kontinuierlichen Ablaufes die eigentliche Sterilisation abschnittsweise durchzuführen und damit die Vorteile der kontinuierlichen mit denen der diskontinuierlichen Verfahrensweise zu verbinden.

Zur Durchführung des neuen Verfahrens wird erfindungsgemäß eine Vorrichtung vorgeschlagen, bei der ein liegender zylindrischer Behältermantel drei in Längsrichtung aufeinanderfolgende Abschnitte umfaßt, nämlich eine Aufgabekammer, in die das aufgeheizte Behandlungsgut eingetragen wird, als Sterilisationsbehälter eine mit ihrem Anfang an die Aufgabekammer angeschlossene, zum Behältermantel konzentrische drehbar angetriebene Rohrwendel und eine an das Ende der Rohrwendel angeschlossene Entleerungskammer, aus der das Behandlungsgut zur Entspannung ausgetragen wird, wobei die zugeführte Menge an Behandlungsgut und die Drehgeschwindigkeit der Rohrwendel so aufeinander abgestimmt sind, daß nur die jeweils unten liegende Hälfte der Rohrwendel mit Behandlungsgut gefüllt ist und der Raum zwischen Rohrwendel und Behältermantel beheizbar ist. Aufgabekammer, Rohrwendel und Entleerungskammer stehen dabei unter gleichem und während der Behandlung gleichbleibendem Druck. Eine solche Ausgestaltung gewährleistet in einfachster Weise sowohl eine zuverlässige Trennung der einzelnen Teilmengen des Behandlungsgutes als auch eine hervorragende Steuerung des Sterilisationsvorganges.

Zur weiteren Verbesserung der Gleichmäßigkeit der Sterilisationsbehandlung des Behandlungsgutes ist vorteilhaft die Rohrwendel mit Einbauten zur verstärkten Umschichtung und Durchmischung des Behandlungsgutes innerhalb der einzelnen Windungen der Rohrwendel versehen und in der Entleerungskammer ein Schöpfrad angeordnet, welches das Behandlungsgut in eine mittige Wanne fördert, aus der es durch ein axial liegendes Rohr ausgetragen wird. Die Ausgestaltung der Entleerungskammer gestattet eine ununterbrochene Übergabe des Behandlungsgutes aus dem Sterilisationsbehälter zur Entspannung und vervollständigt hier die insgesamt kontinuierliche Betriebsweise.

Unter Einbeziehung der Zeichnung werden Ausführungsbeispiele der Erfindung beschrieben.
- Fig. 1: zeigt schematisch den Verfahrensablauf,
- Fig. 2: einen Sterilisationsbehälter im Längsschnitt,
- Fig. 3: eine Entleerungskammer mit Schöpfrad im Querschnitt und
- Fig. 4: schematisiert eine für den Verfahrensablauf geeignete Anlage.

Das Behandlungsgut muß pumpfähig sein. Bei mehrphasigen Medien und Feststoffen mit mehr als 25 bis 30 mm Durchmesser ist eine Zerkleinerung erforderlich.

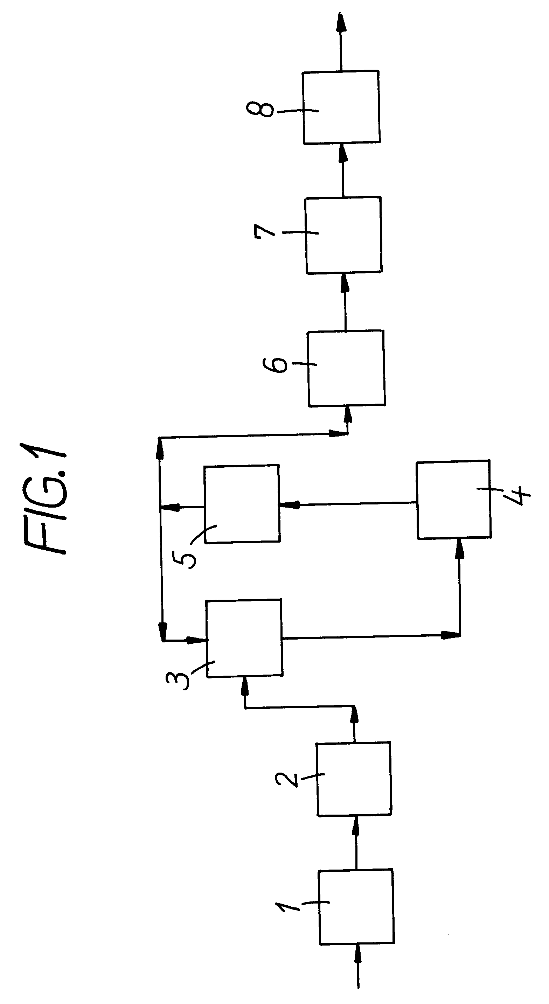

Entsprechend der Darstellung in Fig. 1 wird das Behandlungsgut mittels einer Pumpe 1 auf den erforderlichen Druck gebracht und über eine Vorwärmstufe 2 in einen Vorlagebehälter 3 gepumpt. Die Vorwärmung des Behandlungsgutes in der Vorwärmstufe 2 erfolgt unter Ausnutzung von Abwärme aus dem Sterilisationsprozeß, beispielsweise durch Nutzung des Entspannungsdampfes, der bei der Entspannung des Sterilgutes in einem Entspannungsbehälter 8 anfällt, und/oder durch Nutzung des Heizdampfkondensats aus einem Enderhitzer 5.

Das Behandlungsgut wird in den Vorlagebehälter 3 derart eingegeben, daß dabei die inerten Gase aus dem Behandlungsgut entfernt und kontrolliert aus dem Behälter abgezogen werden. Hierdurch wird gewährleistet, daß die im Behandlungsgut befindlichen und die während der Aufheizung gebildeten oder freigesetzten Inertgase weitestgehend entfernt werden. Die möglicherweise während der anschließenden Sterilisation in geringem Umfang entstehenden Gase wirken sich durch diese Maßnahme nicht mehr nachhaltig auf die Sterilisation aus. Der Vorlagebehälter 3 dient gleichzeitig als Vorlage für eine Pumpe 4, die das Behandlungsgut über den mit Sattdampf beheizten Enderhitzer 5 und den Vorlagebehälter 3 zirkuliert. Dadurch wird eine homogene Aufheizung des zu sterilisierenden Behandlungsgutes auf eine Endtemperatur, die etwa 5 bis 7°C über der Sterilisationstemperatur liegt, erreicht. Durch die Zirkulation werden - besonders bei schlecht wärmeleitenden Medien - lokale Überhitzungen des Behandlungsgutes im wandnahen Bereich minimiert.

Nach dem Enderhitzer 5 wird ein Teilstrom des Behandlungsgutes abgezogen und einem Sterilisationsbehälter 6 zugeführt.

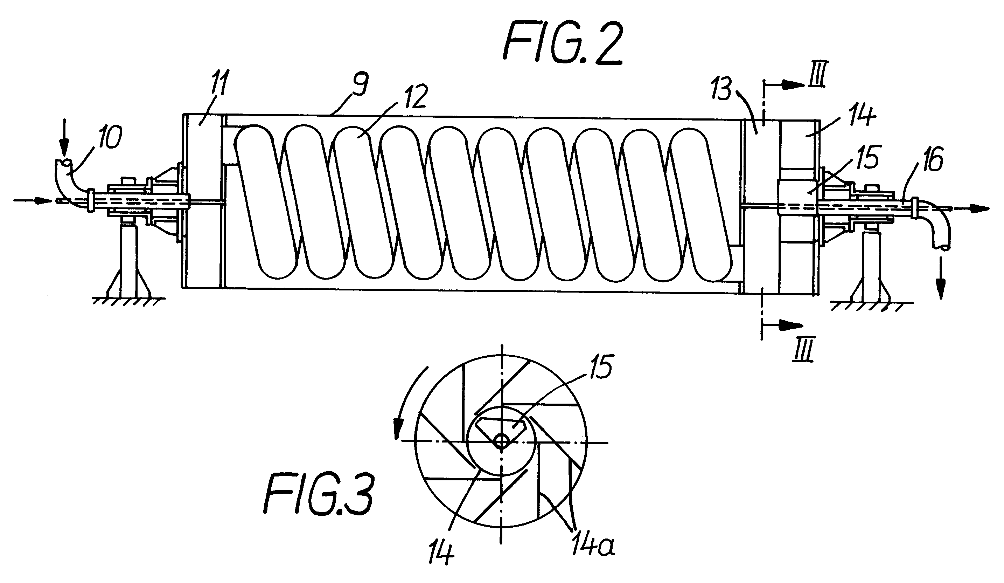

Für den Sterilisationsbehälter 6 ist, wie in Fig. 2 und 3 dargestellt, ein horizontaler zylindrischer Behältermantel 9 vorgesehen, der um seine Längsachse rotiert. An den beiden Zylinderenden sind je eine Aufgabe- und eine Entleerungskammer 11 und 13 angeordnet. Beide Kammern sind durch eine dazwischenliegende horizontale, zylindrische Rohrwendel 12, die mit dem Behälter um ihre Längsachse rotiert und den Sterilisationsbehälter darstellt, miteinander verbunden.

Behältermantel 9 oder Behältermantel 9, Aufgabe- und Entleerungskammer 11 und 13 können auch vorteilhaft stillstehend ausgeführt sein. In letzterem Fall ist die Rohrwendel mit konzentrischen Enden versehen, mit denen sie an die beiden Kammern 11 und 13 angeschlossen ist und die sich zudem für die Drehlagerung eignen.

Durch die Drehung der Rohrwendel 12 wird das zu sterilisierende Behandlungsgut in einer schraubenförmigen Bewegung kontinuierlich von der Aufgabekammer 11 zur Entleerungskammer 13 gefördert (Fig. 2) und von dort mittels eines Schöpfrades 14 mit Schaufeln 14a über eine mittige Wanne 15 (Fig. 3), ein zentrales Ablaufrohr 16 und eine Druckschleuse 7 (Fig. 1) in den Entspannungsbehälter 8 entleert.

Das Behandlungsgut wird nach der Entspannung weiteren Verarbeitungsschritten zugeführt.

Weiterhin geeignet für das erfindungsgemäße Sterilisationsverfahren ist der Sterilisationsbehälter nach Fig. 2, den das Behandlungsgut kontinuierlich durchläuft, wobei jedes Partikel des Behandlungsgutes über eine definierte Zeit einer definierten Temperatur ausgesetzt ist.

Der horizontal angeordnete, um die Längsachse rotierende Behältermantel 9 ist in drei vertikale Abschnitte unterteilt, und zwar
eine Aufgabekammer 11, in die das Behandlungsgut durch ein in der Rotationsachse befindliches Befüllrohr 10 eingespeist wird,
eine liegende, konzentrische, zylindrische Rohrwendel 12 als eigentliche Sterilisationsstrecke, die mit dem Zylindermantel fest verbunden ist und sich mit ihm um die Zylinderachse dreht, sowie
eine Entleerungskammer 13 mit integriertem Schöpfrad 14, axial angeordneter Auffangwanne 15 und axialem Ablaufrohr 16.

Die Aufgabe- und die Entleerungskammer 11 und 13 bilden jeweils eine Zylinderscheibe und sind an den gegenüberliegenden Zylinderenden angeordnet. Sie sind nur durch das Rohrinnere der dazwischenliegenden Rohrwendel 12 miteinander verbunden.

Die Aufgabe- und die Entleerungskammer 11 und 13 sowie die dazwischenliegenden Rohrwendel 12 werden lediglich bis zur Rotationsachse mit Behandlungsgut gefüllt. Durchläuft die aufgabekammerseitige Öffnung der Rohrwendel die untere Kreishälfte, so füllt sich ein halber Wendelgang mit Behandlungsgut. Durchläuft dieselbe Öffnung die obere Kreishälfte, so füllt sich ein halber Wendelgang mit Brüden. Dieser Vorgang wiederholt sich bei jeder Umdrehung. Die jeweiligen Teilmengen des Behandlungsgutes in den unteren Wendelganghälften sind somit durch die jeweils in den oberen Wendelganghälften befindlichen im wesentlichen aus Sattdampf bestehenden Brüden voneinander getrennt. Aufgrund der vorhergehend durchgeführten Entgasung des Behandlungsgutes ist die Dampfphase oberhalb des Feststoff-Flüssigkeits-Gemisches im Inneren des Wendelganges gesättigt. Mikroorganismen werden unter diesen Bedingungen erfahrungsgemäß innerhalb weniger Sekunden abgetötet. Der Dampfraum zwischen zwei benachbarten Behandlungsgutspiegeln stellt daher eine unüberwindliche Barriere dar, die verhindert, daß Mikroorganismen lebend von der Flüssigkeit innerhalb einer Wendel in eine benachbarte gelangen können. Durch die Drehung der Rohrwendel 12 werden so Behandlungsgut und Brüden in jedem Wendelgang kontinuierlich von der Aufgabekammer 11 zur Entleerungskammer 13 transportiert, ohne daß eine Vermischung in den einzelnen Wendelgängen stattfinden kann. Durchläuft das entleerungskammerseitige Ende der Rohrwendel 12 die untere Kreishälfte, so entleert sich dabei die Teilmenge eines Wendelganges in die Entleerungskammer 13. Die Drücke in der Aufgabe- und der Entleerungskammer 11 und 13 sowie der Rohrwendel 12 sind gleich und werden mittels entsprechender Einrichtungen, die die Zuführung von Wasserdampf bzw. die Abführung von Brüden regeln, auf den Siededruck von Wasser bei der Zulauftemperatur des Behandlungsgutes in den Sterilisationsbehälter 9 konstant gehalten. Der Mantelraum um die Rohrwendel 12 ist vorzugsweise mit gesättigtem Heizdampf beaufschlagt. Der Druck des Heizdampfes ist dem Druck in der Aufgabe- und Entleerungskammer 11 und 13 gleich. Dadurch wird die Temperatur über die Sterilisationszeit konstant gehalten sowie die Verdampfung aus der Flüssigphase und die Kondensation aus der Dampfphase in den Wendelgängen verhindert. Somit ist gewährleistet, daß sich über die gesamte Länge des Sterilisationsbehälters der gleiche Gutstand einstellt, was zur Vermeidung der axialen Längsvermischung, d.h. dem unerwünschten Übertreten eines Teils des Behandlungsgutes von einem Wendelgang in den nächsten beiträgt. Durch diese Vorgehensweise ergibt sich weiter, daß sowohl das Behandlungsgut in der unteren Wendelganghälfte als auch der darüberliegende Brüden beim Transport von der Aufgabekammer 11 zur Entleerungskammer 13 eine genau definierte Verweilzeit besitzen und währenddessen einer definierten Temperatur ausgesetzt sind.

Es kann beispielsweise für die Verarbeitung von tierischen Abfällen zu Tierfutter eine Sterilisation des eingehenden Behandlungsgutes bei einer Temperatur von mindestens 133°C und bei einem Absolutdruck von 3 bar vorgegeben werden, wobei die Behandlungsdauer mindestens 20 Minuten betragen muß. Da die Anlagen zur Verarbeitung von tierischen Abfällen in der Regel Rohwarenmengen von ca. 5 bis 15 t/h durchsetzen, ist ein kontinuierliches Sterilisationsverfahren von besonderem Interesse, zumal die in den Anlagen hergestellten Produkte - z.B. tierische Fette und Fleischmehl - nur eine geringe Wertschöpfung aufweisen. Das erfindungsgemäße Verfahren und die erfindungsgemäße Apparatur sind im besonderen Maße geeignet, die Sterilisation unter Einhaltung vergleichbarer Vorgaben wirtschaftlich durchzuführen.

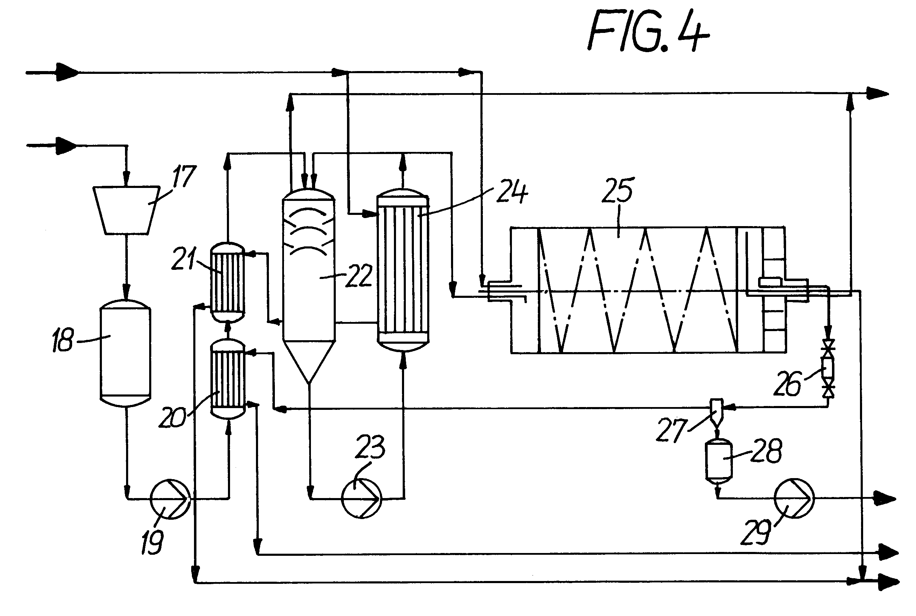

Ein Verfahrensfließbild für eine solche Sterilisationsanlage von tierischen Abfällen bzw. Schlachtnebenprodukten zeigt Fig. 4.

Die angelieferten tierischen Abfälle werden zunächst in Brechern 17 zerkleinert, so daß ein pumpfähiger Fleischbrei mit Feststoffen von etwa 25 bis 30 mm Durchmesser entsteht. Dieser Fleischbrei, dessen natürlicher Wassergehalt etwa 60 bis 70 % (Gewicht) beträgt, wird aus einem Zwischenbehälter 18 mittels einer Pumpe 19 durch zwei Wärmeübertrager 20 und 21 in einen Entgasungsbehälter 22 gefördert. Im ersten Wärmeübertrager 20 wird der Fleischbrei mittels Entspannungsdampf, der bei der Entspannung des Behandlungsgutes hinter der Schleuse 26 entsteht, von ca. 15°C auf ca. 55°C vorgewärmt. Bei der weiteren Vorwärmung des Fleischbreies von ca. 55°C auf 65°C im zweiten Wärmeübertrager 21 wird Restwärme des Heizmediums aus einem Enderhitzer 24 genutzt. Dies kann beispielsweise hochgespanntes Heizdampfkondensat oder Nachdampf aus der Kondensatentspannung sein.

In dem Entgasungsbehälter 22 wird der Fleischbrei von oben aufgegeben und über Einbauten geführt, so daß die im Brei eingeschlossenen Inertgase entweichen können. Diese werden kontrolliert aus dem System abgezogen. Der Entgasungsbehälter 22 dient auch als Pumpvorlage für eine Pumpe 23, die den Fleischbrei durch den Enderhitzer 24 in den Entgasungsbehälter 22 rezirkuliert, wodurch eine weitestgehende Entfernung nichtkondensierbarer Gase aus dem Fleischbrei und eine homogene Erhitzung erreicht werden. Der zirkulierte Massenstrom beträgt in der Regel ein Mehrfaches des zugeführten Fleischbreimassenstromes. Der dem Zirkulationssystem kontinuierlich zugeführte Massenstrom wird hinter dem Enderhitzer, nachdem der Fleischbrei eine Temperatur von 135 bis 140°C erreicht hat, abgezogen und der Aufgabekammer des Sterilisationsbehälters 25 zugeführt. Den Sterilisationsbehälter 25 durchläuft der Fleischbrei in der oben ausführlich beschriebenen Weise. Nach erfolgter Sterilisation wird der Fleischbrei in einer Schleuse 26, die beispielsweise als Doppelschieberschleuse ausgeführt ist, auf Atmosphärendruck entspannt. Die dabei entstehenden Wasserdampfbrüden werden in einem Abscheider 27 vom Fleischbrei abgetrennt und im Wärmeübertrager 20 als Heizmedium genutzt. Der Fleischbrei wird in einem Behälter 28 aufgefangen und mittels einer Pumpe 29 den weiteren Verarbeitungsschritten, beispielsweise Trocknung und Entfettung usw., zugeführt.

## Patentansprüche

1. Verfahren zur Sterilisierung von pumpfähigem Behandlungsgut durch Aufheizen, Durchleiten durch einen beheizten liegenden Sterilisationsbehälter und anschließendes Entspannen und Abkühlen des Behandlungsgutes, dadurch gekennzeichnet, daß das Behandlungsgut zunächst unter mindestens den Sattdampfdruck, bezogen auf seine Sterilisationstemperatur, gesetzt, dann auf mindestens die Sterilisationstemperatur erhitzt und von inerten Gasen befreit und danach über eine ausreichende Sterilisationszeit während der Durchleitung durch den Sterilisationsbehälter (6) auf mindestens der Sterilisationstemperatur gehalten wird.

2. Verfahren nach Anspruch 1, dadurch gekennzeichnet, daß die Aufheizung in zwei Stufen erfolgt, nämlich einer Vorwärmstufe (2), in der anfallende Abwärme genutzt, und einer Enderhitzerstufe (5), in der aus Primärenergie erzeugte Wärme verwendet wird.

3. Verfahren nach Anspruch 2, dadurch gekennzeichnet, daß die Vorwärmung des Behandlungsgutes in zwei Schritten erfolgt, wobei im ersten Schritt bei der Entspannung (8) anfallender Wasserdampf und im zweiten Schritt Abwärme aus der Enderhitzerstufe (5) eingesetzt wird.

4. Verfahren nach Anspruch 2 oder 3, dadurch gekennzeichnet, daß die Enderhitzung (5) in der Weise erfolgt, daß elektrischer Strom unmittelbar durch das Behandlungsgut fließt und dieses infolge seines ohmschen Widerstandes erwärmt.

5. Verfahren nach Anspruch 2 oder 3, dadurch gekennzeichnet, daß das Behandlungsgut aus der Vorwärmstufe (2) kontinuierlich in einen Vorlagebehälter gegeben und von dort mittels einer Pumpe über den Enderhitzer im Kreislauf zum Vorlagebehälter (3) zurückgeführt wird, wobei im Vorlagebehälter (3) die Entgasung erfolgt.

6. Verfahren nach Anspruch 4, dadurch gekennzeichnet, daß aus dem Kreislauf zwischen Enderhitzer (5) und Vorlagebehälter (3) kontinuierlich ein Teilstrom des Behandlungsgutes abgezogen und dem Sterilisationsbehälter (6) zugeführt wird, der dem aus der Vorwärmstufe (2) in den Kreislauf eingeführten Mengenstrom entspricht, wobei der im Kreislauf geführte Mengenstrom ein Mehrfaches hiervon beträgt.

7. Verfahren nach den Ansprüchen 4 oder 5, dadurch gekennzeichnet, daß die Entgasung in der Weise erfolgt, daß im Gasraum des Vorlagebehälters (3) eine wasserdampfgesättigte Atmosphäre vorliegt.

8. Verfahren nach einem der vorhergehenden Ansprüche, dadurch gekennzeichnet, daß der Durchlauf des Behandlungsgutes durch den Sterilisationsbehälter (6) in mehreren in Durchlaufrichtung aufeinanderfolgenden Teilmengen erfolgt, die je voneinander durch gesättigten Wasserdampf getrennt sind.

9. Vorrichtung zur Durchführung des Verfahrens nach einem der vorhergehenden Ansprüche, dadurch gekennzeichnet, daß ein liegender zylindrischer Behältermantel (9) drei in Längsrichtung aufeinanderfolgende Abschnitte umfaßt, nämlich eine Aufgabekammer (11), in die das aufgeheizte Behandlungsgut eingetragen wird, als Sterilisationsbehälter (6) eine mit ihrem Anfang an die Aufgabekammer (11) angeschlossene, zum Behältermantel (9) konzentrische drehbar angetriebene Rohrwendel (12) und eine an das Ende der Rohrwendel (12) angeschlossene Entleerungskammer (13), aus der das Behandlungsgut zur Entspannung ausgetragen wird, wobei die zugeführte Menge an Behandlungsgut und die Drehgeschwindigkeit der Rohrwendel (12) so aufeinander abgestimmt sind, daß nur die jeweils unten liegende Hälfte der Rohrwendel (12) mit Behandlungsgut gefüllt ist, und der Raum zwischen Rohrwendel (12) und Behältermantel (9) beheizbar ist.

10. Vorrichtung nach Anspruch 9, dadurch gekennzeichnet, daß die Rohrwendel (12) mit Einbauten zur verstärkten Umschichtung und Durchmischung des Behandlungsgutes innerhalb der einzelnen Windungen der Rohrwendel (12) versehen ist und daß in der Entleerungskammer (13) ein Schöpfrad (14) angeordnet ist, welches das Behandlungsgut in eine mittige Wanne (15) fördert, aus der es durch ein axial liegendes Rohr (16) ausgetragen wird.
